# EUROPEAN PATENT APPLICATION

(11) **EP 4 534 127 A1**
(43) Date of publication of application: **09.04.2025**
(21) Application number: 23202197.2
(22) Date of filing: 06.10.2023
(51) Int. Cl.: A61M 16/00

(54) **AIRFLOW DEVICE COMPRISING AIR FLOW PATH AND ACOUSTIC ABSORBER**

(71) Applicant: Koninklijke Philips N.V., 5656 AG Eindhoven (NL)
(72) Inventor: JOUSMA, Hendrik Richard, Eindhoven (NL); BOONSTRA, Bonne Lambert, Eindhoven (NL); SCHALLIG, Michiel, 5656AG Eindhoven (NL); OUWELTJES, Okke, Eindhoven (NL); PASTOORS, Marc Alexander, Eindhoven (NL); DUINEVELD, Paulus Cornelis, Eindhoven (NL); STEUNENBERG, Roelof, Eindhoven (NL)
(74) Representative: Philips Intellectual Property & Standards

(57) **Abstract**

Provided is an airflow device (100), for example a respiratory support device (100), comprising a flow path (102) along which air is displaceable, and an acoustic absorber (104) arranged along at least part of the flow path. Cavities are defined in a material of the acoustic absorber for absorbing sound. The airflow device also comprises an acoustically transparent layer (106) between the flow path and the acoustic absorber. The acoustically transparent layer is configured to transmit sound in the flow path to the acoustic absorber to enable the acoustic absorber to absorb the sound. The acoustically transparent layer has an exterior surface for exposing to the air in the flow path, and an interior surface facing away from the flow path and towards the acoustic absorber.

## Description

### FIELD OF THE INVENTION

The present invention relates to an airflow device, in particular a respiratory support device, that includes an acoustic absorber for absorbing sound generated by the device. The invention further relates to a method for manufacturing such a device.

### BACKGROUND OF THE INVENTION

Airflow devices, such as respiratory support devices, may generate sound during operation. This sound can disturb user(s) of such devices. Sound disturbance can be particularly problematic in the case of respiratory support devices, such as continuous positive airway pressure (CPAP) devices, because of the risk of the user, in other words patient or subject, being unable to sleep due to the sound generated by such devices. This is particularly undesirable when the respiratory support device is being used to treat sleep apnea.

For this reason, acoustic absorption may be implemented in airflow devices, such as CPAP devices, in various ways. For example, foam may be used to absorb sound.

Various challenges nonetheless remain in terms of enhancing and facilitating acoustic absorption in airflow devices, and in particular in respiratory support devices.

### SUMMARY OF THE INVENTION

The invention is defined by the claims.

According to examples in accordance with an aspect of the invention, there is provided a respiratory support device comprising: a flow path along which air is deliverable towards a user of the respiratory support device; an acoustic absorber arranged along at least part of the flow path, cavities being defined in material of the acoustic absorber for absorbing sound; and an acoustically transparent layer between the flow path and the acoustic absorber, the acoustically transparent layer being configured to permit sound in the flow path to be transmitted through the acoustically transparent layer to the acoustic absorber to enable the acoustic absorber to absorb the sound, the acoustically transparent layer having an exterior surface for exposing to the air in the flow path, and an interior surface facing away from the flow path and towards the acoustic absorber.

By arranging the acoustic absorber along at least part of the respiratory support device's flow path, the acoustic absorber can assist to minimize sound being carried along the flow path to the exterior of the respiratory support device.

To this end, cavities are defined in the material of the acoustic absorber to absorb sound. It is noted that some manufacturers of materials for acoustic absorbers label such materials with "pores per inch PPI" as a measure for the number of cavities.

The acoustic absorber may generally be regarded as a sink for soundwaves, which converts sound energy into thermal energy.

Thus, the acoustic absorber can help to minimize the risk of the user of the respiratory support device being disturbed, e.g. woken up, by sound emanating from inside the respiratory support device.

The present invention is partly based on the insight that one or more aspects of performance of a respiratory support device can be enhanced by arranging an acoustically transparent layer between the acoustic absorber and the flow path.

By the acoustically transparent layer being between the acoustic absorber and the flow path, the acoustically transparent layer may assist to minimize or prevent collection of contaminants in and/or on the material of the acoustic absorber that may otherwise accumulate in the cavities of the acoustic absorber and reduce effectiveness of the acoustic absorber during use of the respiratory support device.

Even though the respiratory support device may include an inlet filter, some particles may pass through the inlet filter during use and may risk accumulating in the cavities of the acoustic absorber. Also, some users may remove the inlet filter and use the respiratory support device without the inlet filter. For example, the user may forget to replace the inlet filter after cleaning the inlet filter, or the inlet filter may be damaged without the user noticing. In such scenarios, the risk of contaminants accumulating in the cavities of the acoustic absorber and reducing the effectiveness of the acoustic absorber may be lessened by inclusion of the acoustically transparent layer.

It is noted that the term "acoustically transparent layer" is a term of the art, and refers to a layer that permits sound transfer therethrough.

It is also noted that the term "sound energy", "sound" and "acoustic" as used herein may refer to vibrations that can heard by human beings. Thus, these terms may refer to waves that have frequencies lying between 20 Hz and 20 kHz.

In some embodiments, the acoustically transparent layer is arranged to seal off the acoustic absorber from the air in the flow path. Such sealing off of the acoustic absorber from the air in the flow path may mean that fluid communication, if any, between the flow path and the acoustic absorber is through the acoustically transparent layer.

In this manner, air in the flow path may be prevented from directly contacting the acoustic absorber, with concomitant lessening of the risk of contamination of the acoustic absorber by contaminants in the air.

In some embodiments, the respiratory support device comprises a housing to which the acoustically transparent layer is attached to define a compartment that is sealed off from the air in the flow path, with the acoustic absorber being arranged in the compartment.

Such attachment of the acoustically transparent layer to the housing to define the compartment for receiving the acoustic absorber may assist to minimize the risk of contamination of the acoustic absorber whilst providing a relatively straightforwardly implementable way of mounting the acoustic absorber and the acoustically transparent layer in the respiratory support device.

Implicit in the defining of the compartment by attachment of the acoustically transparent layer to the housing is that the acoustically transparent layer is sealingly attached to the housing.

Such sealing attachment may be implemented in any suitable manner, for example by forming the acoustically transparent layer on the housing in a molding process, welding the acoustically transparent layer and the housing to each other, and/or using an adhesive to adhere the acoustically transparent layer and the housing to each other.

It is noted that the housing can be formed from any suitable material. In some embodiments, the housing is formed from a thermoplastic, e.g. polypropylene.

Alternatively or additionally, the acoustically transparent layer may be formed from a thermoplastic, e.g. polyethylene.

The acoustically transparent layer may comprise a polymer film. Such a polymer film may provide a relatively simple and cost-effective way of providing the acoustically transparent layer.

The polymer film may be apertureless.

Thus, the polymer film may prevent particles in the air in the flow path from reaching the acoustic absorber through the polymer film.

In some embodiments, the acoustically transparent layer comprises a polyethylene film and/or a polyimide film.

The polymer film may be formed in any suitable manner. In some embodiments, the polymer film, e.g. polyimide film, is formed from a two-component epoxy.

The acoustic absorber may have a pitted external surface, for example with at least part of the pitted external surface facing the interior surface of the acoustically transparent layer.

The term "pitted" is intended to mean that the external surface of the acoustic absorber is provided with holes and/or recesses.

In such embodiments, the acoustically transparent layer may assist to alleviate contamination of the holes and/or recesses of the pitted external surface. This alleviation of contamination may, in turn, lead to enhanced acoustic performance of the respiratory support device.

In some embodiments, the acoustic absorber comprises a porous material and/or a perforate member. The perforate member may, for example, include a Helmholtz resonator. In a Helmholtz resonator, the cavities are formed by a plurality of holes defined in a substrate, with the holes facing the acoustically transparent layer.

Air contained in the plurality of holes may oscillate in front of the acoustically transparent layer, and thereby absorb sound transmitted from the flow path via the acoustically transparent layer.

As an alternative or in addition to the perforate member, the acoustic absorber may include a porous material, such as a foam and/or a fibrous material.

In the case of the fibrous material, the cavities in the acoustic absorber's material may be (at least partly) defined between fibers of the fibrous material.

The fibrous material may include fibrous mineral wool, in other words rock wool, and/or glass fiber wool.

Such fibrous materials may provide effective sound absorption. Moreover, the acoustically transparent layer may minimize or prevent fibers from such materials entering the flow path.

In embodiments in which foam is included in, e.g. defines, the material of the acoustic absorber, the foam can provide particularly effective sound suppression. Whilst foams can have an intrinsic odor, e.g. derived from the manner in which they are manufactured, the acoustically transparent layer may assist to minimize the risk of this odor being carried along the flow path towards the user.

The foam can be of any suitable type and made from any suitable material capable of being formed into a foam. In some embodiments, the foam is a silicone foam.

In some embodiments, the interior surface of the acoustically transparent layer contacts the acoustic absorber. Alternatively, a gap may be defined between the interior surface of the acoustically transparent layer and the acoustic absorber. Thus, sound in the flow path may be transmitted to the acoustic absorber via the acoustically transparent layer and by gas, e.g. air, in the gap between the acoustically transparent layer's interior surface and the acoustic absorber.

The respiratory support device may include an air inlet through which ambient air is receivable into the flow path, and a blower unit for displacing the air along the flow path towards the user.

In such embodiments, the acoustic absorber and the acoustically transparent layer may be arranged along the flow path between the air inlet and the blower unit. Thus, the acoustic absorber may absorb sound that would otherwise emanate from the air inlet, due to sound travelling in the opposite direction to flow along the flow path.

In some embodiments, the respiratory support device comprises a filter unit in which one or more air filters is or are mountable. Such a filter unit may, for example, be arranged at the air inlet. This may enable the air filter(s), in other words inlet air filter(s), to filter the ambient air upstream of the acoustically transparent layer. Operating lifetime of the acoustically transparent layer may be prolonged, since the acoustically transparent layer may be less prone to being contaminated by ambient air received in the flow path.

It is generally noted that the respiratory support device may be of any type. In some embodiments, the respiratory support device comprises a continuous positive airway pressure, CPAP, device.

According to another aspect there is provided a method of manufacturing a respiratory support device comprising: arranging an acoustic absorber along at least part of a flow path defined in the device for delivery of air towards a user of the manufactured device, cavities being defined in material of the acoustic absorber for absorbing sound; and arranging an acoustically transparent layer so that the acoustically transparent layer is able to separate the acoustic absorber from air in the flow path, the acoustically transparent layer being configured to permit sound in the flow path to be transmitted through the acoustically transparent layer to the acoustic absorber to enable the acoustic absorber to absorb the sound, the acoustically transparent layer having an exterior surface for exposing to the air in the flow path, and an interior surface for facing away from the flow path and towards the acoustic absorber.

In some embodiments, the method comprises using the acoustically transparent layer to seal off the acoustic absorber from the air in the flow path.

Alternatively or additionally, the method may comprise attaching the acoustically transparent layer to a housing of the respiratory support device to define a compartment that is sealed off from the air in the flow path; the acoustic absorber being arranged in the compartment.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
FIG. 1 provides a perspective interior view of part of an airflow device in the form of a respiratory support device;
FIG. 2 provides a cutaway view of the device shown in FIG. 1;
FIG. 3 provides a schematic depiction of a device according to an example; and
FIG. 4 provides graphs of sound power vs. pressure setting for respiratory support devices having various acoustic absorption arrangements.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Provided is an airflow device, for example a respiratory support device, comprising a flow path along which air is displaceable, and an acoustic absorber arranged along at least part of the flow path. Cavities are defined in a material of the acoustic absorber for absorbing sound. The airflow device also comprises an acoustically transparent layer between the flow path and the acoustic absorber. The acoustically transparent layer is configured to transmit sound in the flow path to the acoustic absorber to enable the acoustic absorber to absorb the sound. The acoustically transparent layer has an exterior surface for exposing to the air in the flow path, and an interior surface facing away from the flow path and towards the acoustic absorber.

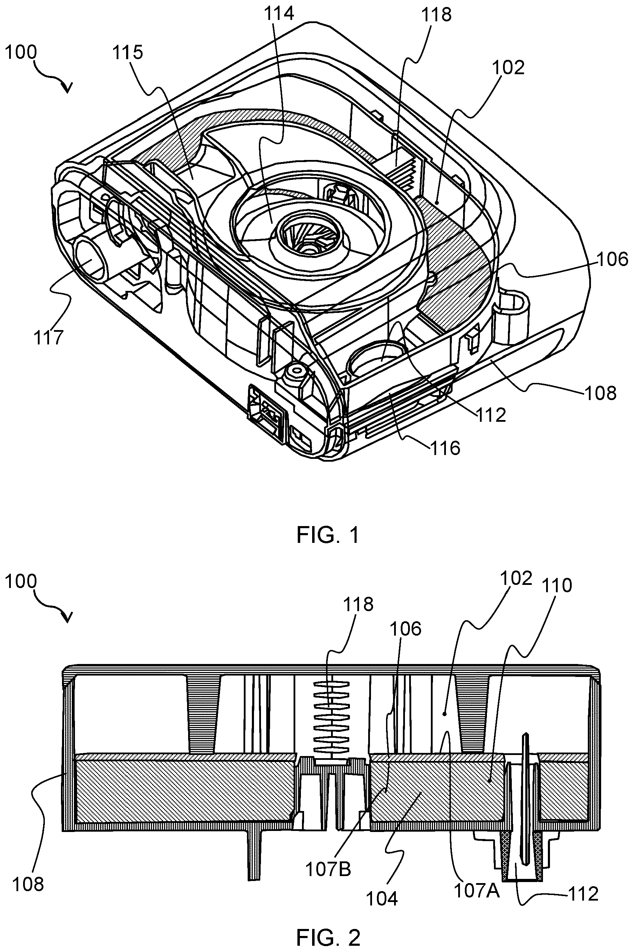

FIG. 1 provides an interior view of an airflow device 100 according to an example. In this example, the airflow device 100 is in the form of a respiratory support device 100 configured to support a user's breathing. In some embodiments, such as shown in FIG. 1, the respiratory support device 100 comprises, e.g. is, a continuous positive airway pressure (CPAP) device 100. Such a CPAP device 100 may be employed, for example, to treat a user's sleep apnea. Alternatively or additionally, the respiratory support device 100 may comprise a bilevel positive airway pressure (BIPAP) device 100, an average volume assured pressure support (AVAPS) device 100, and/or any hospital or home ventilator device 100.

It is noted that the principles of the present disclosure can also be applied to other types of airflow device 100, such as air purification and/or air conditioning devices. In other words, the airflow device 100 may comprise, e.g. be, an air purification and/or air conditioning device.

The airflow device 100 generally comprises a flow path 102 along which air is displaceable. In embodiments in which the airflow device 100 is in the form of a respiratory support device 100, air is deliverable along the flow path 102 towards the user of the respiratory support device 100.

In embodiments in which the airflow device 100 is an air purification and/or air conditioning device, air may be displaceable along the flow path 102 towards a space being supplied with purified and/or conditioned air by the air purification and/or air conditioning device.

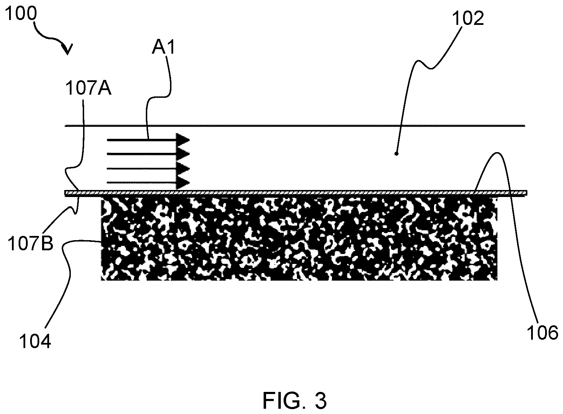

Referring to FIGs. 2 and 3, the airflow device 100 comprises an acoustic absorber 104 arranged along at least part of the flow path 102. The acoustic absorber 104 may generally be regarded as a sink for soundwaves, which converts sound energy into thermal energy. Cavities are defined in material of the acoustic absorber 104 for absorbing sound. It is noted that some manufacturers of materials for acoustic absorbers label such materials with "pores per inch PPI" as a measure for the number of cavities in the material of the acoustic absorber 104.

The cavities defined in the material of the acoustic absorber 104 may mean that the acoustic absorber 104 has a pitted external surface, in other words an external surface provided with holes and/or recesses.

The acoustic absorber 104 can help to minimize the risk of user(s) of the airflow device 100 being disturbed or discomforted by sound emanating from inside the airflow device 100. In embodiments in which the airflow device 100 comprises a respiratory support device 100, the acoustic absorber 104 may assist to reduce the risk of the user, e.g. a sleep apnea sufferer, being disturbed, e.g. woken up, by sound emanating from inside the airflow device 100.

The acoustic absorber 104 can have any suitable design provided that the acoustic absorber 104 is capable of acting as a sink for soundwaves. In some embodiments, the acoustic absorber 104 comprises a porous material and/or a perforate member. The perforate member may, for example, include a Helmholtz resonator. In a Helmholtz resonator, the cavities are formed by a plurality of holes defined in a substrate, with the holes facing the flow path 102. Air contained in the plurality of holes may oscillate and thereby absorb sound transmitted from the flow path 102.

As an alternative or in addition to the perforate member, the acoustic absorber 104 may include a porous material, such as a foam and/or a fibrous material.

In the case of the fibrous material, the cavities in the acoustic absorber's 104 material, as well as the pitted external surface of the acoustic absorber 104, may be (at least partly) defined between fibers of the fibrous material. The fibrous material may include fibrous mineral wool, in other words rock wool, and/or glass fiber wool. Such fibrous materials may provide effective sound absorption.

In embodiments in which foam is included in, e.g. defines, the material of the acoustic absorber 104, the foam can provide particularly effective sound suppression. The foam can be of any suitable type and made from any suitable material capable of being formed into a foam. In some embodiments, the foam is a silicone foam.

Irrespective of the particular type of acoustic absorber 104 included in the airflow device 100, the airflow device 100 may include, in addition to the acoustic absorber 104, an acoustically transparent layer 106 between the flow path 102 and the acoustic absorber 104. The acoustically transparent layer 106 is configured to permit sound in the flow path 102 to be transmitted through the acoustically transparent layer 106 to the acoustic absorber 104 to enable the acoustic absorber 104 to absorb the sound.

With continued reference to FIGs. 2 and 3, the acoustically transparent layer 106 has an exterior surface 107A exposed to the air in the flow path 102, and an interior surface 107B facing away from the flow path 102 and towards the acoustic absorber 104. One or more aspects of performance of the airflow device 100 can be enhanced by arranging such an acoustically transparent layer 106 between the acoustic absorber 104 and the flow path 102. In particular, by the acoustically transparent layer 106 being interposed between the acoustic absorber 104 and the flow path 102, the acoustically transparent layer 106 may assist to minimize or prevent collection of contaminants in and/or on the material of the acoustic absorber 104 that may otherwise accumulate in the cavities of the acoustic absorber 104 and reduce effectiveness of the acoustic absorber 104 during use of the airflow device 100.

For example, the acoustically transparent layer 106 may assist to alleviate contamination of the holes and/or recesses of the pitted external surface of the acoustic absorber 104 during use of the airflow device 100.

In some embodiments, such as shown in FIGs. 2 and 3, the interior surface 107B of the acoustically transparent layer 106 contacts the acoustic absorber 104. In other embodiments (not shown), a gap may be defined between the interior surface 107B of the acoustically transparent layer 106 and the acoustic absorber 104. Thus, sound in the flow path 102 may be transmitted to the acoustic absorber 104 via the acoustically transparent layer 106 and by gas, e.g. air, in the gap between the acoustically transparent layer's 106 interior surface 107B and the acoustic absorber 104.

In embodiments in which the acoustic absorber 104 includes a fibrous material, such as fibrous mineral wool and/or glass fiber wool, the acoustically transparent layer 106 may minimize or prevent fibers from such materials entering the flow path 102. In embodiments in which foam is included in, e.g. defines, the material of the acoustic absorber 104, the acoustically transparent layer 106 may assist to minimize the risk of odor intrinsic to the foam, e.g. derived from manufacture of the foam, being carried along the flow path 102 towards the user.

The acoustically transparent layer 106 can be formed from any suitable material and fabricated in any suitable manner provided that the acoustically transparent layer 106 is capable of minimizing or preventing collection of contaminants in and/or on the material of the acoustic absorber 104, while permitting sound in the flow path 102 to be transmitted through the acoustically transparent layer 106 to the acoustic absorber 104.

The acoustically transparent layer 106 may be sufficiently thin and flexible to let soundwaves pass therethrough.

In other words, the acoustically transparent layer 106 may be made from a thin, flexible material placed between the acoustic absorber 104, e.g. acoustically absorbing foam, and the flow path 102, e.g. a patient airway path 102 along which air is delivered to a patient when the airflow device 100 is in the form of a respiratory support device 100.

In some embodiments, the acoustically transparent layer 106 comprises a polymer film, such as a polyethylene film or a polyimide film. Such a polymer film may provide a relatively simple and cost-effective way of providing the acoustically transparent layer 106.

The polymer film may be apertureless. Thus, the polymer film may prevent particles in the air in the flow path 102 from reaching the acoustic absorber 104 through the polymer film.

The polymer film, e.g. polyimide film, may, for instance, be formed from a two-component epoxy. For example, the acoustically transparent layer 106 may be formed by covering an acoustic absorber 104, e.g. an acoustically absorbing foam, with such a two-component epoxy that cures to form the acoustically transparent layer 106 on the external surface of the acoustic absorber 104.

In some embodiments, and as best shown in FIG. 2, the acoustically transparent layer 106 is arranged to seal off the acoustic absorber 104 from the air in the flow path 102. Such sealing off of the acoustic absorber 104 from the air in the flow path 102 may mean that fluid communication, if any, between the flow path 102 and the acoustic absorber 104 is through the acoustically transparent layer 106. In this manner, air in the flow path 102 may be prevented from directly contacting the acoustic absorber 104, with concomitant lessening of the risk of contamination of the acoustic absorber 104 by contaminants in the air.

The acoustically transparent layer 106 preferably separates the acoustic absorber 104, e.g. foam, from the flow path 102 such that the acoustic absorber 104 is not in fluid contact with the air in the flow path 102.

The sealing off of the acoustic absorber 104 may mean that the acoustically transparent layer 106 provides an airtight barrier that seals the acoustic absorber 104 from contact with air in the flow path 102, e.g. the patient airstream in the case of the airflow device 100 being a respiratory support device 100.

For example, the acoustically transparent layer 106 may provide a 100% seal, e.g. by being sealingly attached to the acoustic absorber 104 and/or to the material, e.g. housing material, that defines the flow path 102.

With continued reference to FIG. 2, the airflow device 100 may comprise a housing 108 to which the acoustically transparent layer 106 is attached to define a compartment 110 that is sealed off from the air in the flow path 102. In such embodiments, the acoustic absorber 104 may be arranged in the compartment 110.

The housing 108 can be formed from any suitable material. In some embodiments, the housing 108 is formed from a thermoplastic, e.g. polypropylene.

Attachment of the acoustically transparent layer 106 to the housing 108 to define the compartment 110 for receiving the acoustic absorber 104 may assist to minimize the risk of contamination of the acoustic absorber 104 whilst providing a relatively straightforwardly implementable way of mounting the acoustic absorber 104 and the acoustically transparent layer 106 in the airflow device 100.

Implicit in the defining of the compartment 110 by attachment of the acoustically transparent layer 106 to the housing 108 is that the acoustically transparent layer 106 is sealingly attached to the housing 108. Such sealing attachment may be implemented in any suitable manner, for example by forming the acoustically transparent layer 106 on the housing 108 in a molding process, welding the acoustically transparent layer 106 and the housing 108 to each other, and/or using an adhesive to adhere the acoustically transparent layer 106 and the housing 108 to each other.

Referring again to FIG. 1, the airflow device 100 may include an air inlet 112, e.g. an air inlet 112 delimited by the housing 108, through which ambient air is receivable into the flow path 102.

The airflow device 100 may also include a blower unit 114 for displacing the air along the flow path 102 towards the user or, as the case may be, towards the space to which purified and/or conditioned air is delivered by the airflow device 100.

The acoustic absorber 104 and the acoustically transparent layer 106 may be arranged along the flow path 102 (at least) between the air inlet 112 and the blower unit 114, for example between the air inlet 112 and a blower inlet 115 of the blower unit 114. Thus, the acoustic absorber 104 may absorb sound that would otherwise emanate from the air inlet 112, due to sound travelling in the opposite direction to the direction of flow along the flow path 102. The direction of airflow along the flow path is denoted by the arrows A1 in FIG. 3.

In some embodiments, and referring again to FIG. 1, the airflow device 100 comprises a filter unit in which one or more air filters 116 is or are mountable. Such a filter unit may, for example, be arranged at the air inlet 112. This may enable the air filter(s) 116, in other words inlet air filter(s) 116, to filter the ambient air upstream of the acoustically transparent layer 106. Operating lifetime of the acoustically transparent layer 106 may be prolonged, since the acoustically transparent layer 106 may be less prone to being contaminated by ambient air received in the flow path 102.

It is generally noted that some particles may pass through the inlet filter 116 during use and may risk accumulating in the cavities of the acoustic absorber 104. Also, some users may remove the inlet filter(s) 116 and use the airflow device 100 without the inlet filter(s) 116. For example, the user may forget to replace the inlet filter(s) 116 after cleaning the inlet filter(s) 116, or the inlet filter(s) 116 may be damaged without the user noticing. In such scenarios, the risk of contaminants accumulating in the cavities of the acoustic absorber 104 and reducing the effectiveness of the acoustic absorber 104 may be lessened by inclusion of the acoustically transparent layer 106.

The blower unit 114 may have any suitable design provided that the blower unit 114 is capable of displacing air along the flow path 102. The blower unit 114 may include a motorized fan that receives air via the blower inlet 115 and forces the air downstream via a blower outlet 117.

In embodiments in which the airflow device 100 is in the form of a respiratory support device 100, a humification unit (not visible) may be arranged downstream of the blower outlet 117.

In some embodiments, such as shown in FIGs. 1 and 2, the airflow device 100 comprises an airflow sensor 118 for sensing airflow in the flow path 102, e.g. between the air inlet 112 and the blower unit 114.

In embodiments in which the air flow device 100 is a respiratory support device 100, the airflow sensor 118 may fulfil a process control and/or safety function that ensures that the requisite airflow is provided to the user to support his/her breathing.

The present disclosure also provides a method for manufacturing the airflow device 100, e.g. the airflow device in the form of a respiratory support device 100.

The method comprises arranging an acoustic absorber 104 along at least part of a flow path 102 defined in the device 100 for delivery of air. Cavities may be defined in material of the acoustic absorber 104 for absorbing sound, as previously described.

The method also comprises arranging an acoustically transparent layer 106 so that the acoustically transparent layer 106 is able to separate the acoustic absorber 104 from air in the flow path 102. The acoustically transparent layer 106 permits sound in the flow path 102 to be transmitted through the acoustically transparent layer 106 to the acoustic absorber 104 to enable the acoustic absorber 104 to absorb the sound, as also described above.

The acoustically transparent layer 106 has an exterior surface 107A for exposing to the air in the flow path 102, and an interior surface 107B for facing away from the flow path 102 and towards the acoustic absorber 104, e.g. towards a pitted external surface of the acoustic absorber 104.

In some embodiments, the method comprises using the acoustically transparent layer 106 to seal off the acoustic absorber 104 from the air in the flow path 102.

The method may comprise attaching the acoustically transparent layer 106 to a housing 108 of the airflow device 100 to define a compartment 110 that is sealed off from the air in the flow path 102; the acoustic absorber 104 being arranged in the compartment 110.

### Experimental examples

Use of the acoustically transparent layer 106 was experimentally investigated by constructing a prototype airflow device 100 based on a Philips DreamStation^{®}. The housing 108 of the so-called blower box of the DreamStation^{®} was opened and the acoustically absorbing foam arranged along the DreamStation^{®}'s flow path 102 was covered with a thin sheet of polyimide film (Kapton^{®} from DuPont) cut in the same shape as the foam. It is noted that the acoustically absorbing foam in this non-limiting example was silicone foam.

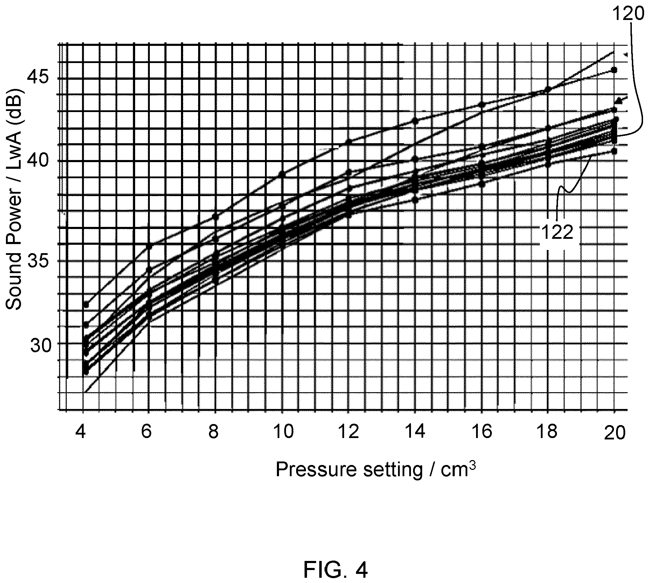

FIG. 4 provides graphs of sound power vs. pressure setting for respiratory support devices 100 of the type shown in FIGs. 1 and 2 having various acoustic absorption arrangements. Graph 120 corresponds to the Philips DreamStation^{®} without the above-described adaptation to include the acoustically transparent layer 106. Graph 122 corresponds to the above-described prototype that includes the acoustically transparent layer 106. Comparison of graphs 120 and 122 indicates that the prototype performed well, with the airflow device 100 according to the present disclosure exhibiting lower noise levels relative to a comparable device that does not include the acoustically transparent layer 106.

Without wishing to be bound by any particular theory, the acoustically transparent layer 106 may, as well as avoiding contamination of the acoustic absorber 104, assist to reduce turbulence associated with interaction between air in the flow path 102 with the pitted external surface of the acoustic absorber 104. This alleviation of turbulence may contribute to enhanced acoustic performance of the airflow device 100.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A respiratory support device (100) comprising:
a flow path (102) along which air is deliverable towards a user of the respiratory support device;
an acoustic absorber (104) arranged along at least part of the flow path, cavities being defined in material of the acoustic absorber for absorbing sound; and
an acoustically transparent layer (106) between the flow path and the acoustic absorber, the acoustically transparent layer being configured to permit sound in the flow path to be transmitted through the acoustically transparent layer to the acoustic absorber to enable the acoustic absorber to absorb the sound, the acoustically transparent layer having an exterior surface (107A) for exposing to the air in the flow path, and an interior surface (107B) facing away from the flow path and towards the acoustic absorber.

2. The respiratory support device (100) according to claim 1, wherein the acoustically transparent layer (106) is arranged to seal off the acoustic absorber (104) from the air in the flow path (102).

3. The respiratory support device (100) according to claim 1 or claim 2, comprising a housing (108), wherein the acoustically transparent layer (106) is attached to the housing to define a compartment (110) that is sealed off from the air in the flow path, the acoustic absorber (104) being arranged in the compartment.

4. The respiratory support device (100) according to any one of claims 1 to 3, wherein the acoustically transparent layer (106) comprises a polymer film.

5. The respiratory support device (100) according to any one of claims 1 to 4, wherein the acoustically transparent layer (106) comprises a polyethylene film and/or a polyimide film.

6. The respiratory support device (100) according to any one of claims 1 to 5, wherein the acoustic absorber (104) comprises a porous material and/or a perforate member.

7. The respiratory support device (100) according to any one of claims 1 to 6, wherein the acoustic absorber (104) comprises foam.

8. The respiratory support device (100) according to any one of claims 1 to 7, wherein the interior surface (107B) of the acoustically transparent layer (106) contacts the acoustic absorber (104).

9. The respiratory support device (100) according to any one of claims 1 to 7, wherein a gap is defined between the interior surface (107B) of the acoustically transparent layer (106) and the acoustic absorber (104).

10. The respiratory support device (100) according to any one of claims 1 to 9, comprising an air inlet (112) through which ambient air is receivable into the flow path (102), and a blower unit (114) for displacing the air along the flow path towards the user.

11. The respiratory support device (100) according to claim 10, wherein the acoustic absorber (104) and the acoustically transparent layer (106) are arranged along the flow path (102) between the air inlet (112) and the blower unit (114).

12. The respiratory support device (100) according to any one of claims 1 to 11, comprising a filter unit in which one or more air filters (116) is or are mountable.

13. The respiratory support device (100) according to any one of claims 1 to 12, comprising a continuous positive airway pressure, CPAP, device.

14. A method of manufacturing a respiratory support device (100) comprising:
arranging an acoustic absorber (104) along at least part of a flow path (102) defined in the device for delivery of air towards a user of the manufactured device, cavities being defined in material of the acoustic absorber for absorbing sound; and
arranging an acoustically transparent layer (106) so that the acoustically transparent layer is able to separate the acoustic absorber from air in the flow path, the acoustically transparent layer being configured to permit sound in the flow path to be transmitted through the acoustically transparent layer to the acoustic absorber to enable the acoustic absorber to absorb the sound, the acoustically transparent layer having an exterior surface for exposing to the air in the flow path, and an interior surface for facing away from the flow path and towards the acoustic absorber.

15. The method according to claim 14, comprising using the acoustically transparent layer (106) to seal off the acoustic absorber (104) from the air in the flow path (102); and/or attaching the acoustically transparent layer to a housing of the respiratory support device (100) to define a compartment (110) that is sealed off from the air in the flow path, the acoustic absorber being arranged in the compartment.
